(19) **Europäisches Patentamt / European Patent Office / Office européen des brevets**

(11) **EP 3 406 232 B1**

(12) **EUROPEAN PATENT SPECIFICATION**

(45) Date of publication and mention
of the grant of the patent:
**11.12.2019 Bulletin 2019/50**

(51) Int Cl.:
*A61F 13/15* (2006.01)          *A61F 13/494* (2006.01)
*A61F 13/475* (2006.01)          *A61F 13/49* (2006.01)

(21) Application number: **16891552.8**

(86) International application number:
**PCT/JP2016/056171**

(22) Date of filing: **01.03.2016**

(87) International publication number:
**WO 2017/145395 (31.08.2017 Gazette 2017/35)**

(54) **DISPOSABLE DIAPER**

WEGWERFWINDEL

COUCHE JETABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2016 JP 2016035693**

(43) Date of publication of application:
**28.11.2018 Bulletin 2018/48**

(73) Proprietor: **Unicharm Corporation**
**Ehime 799-0111 (JP)**

(72) Inventors:
• **HASHIMOTO, Tatsuya**
**Kanonji-shi**
**Kagawa 769-1602 (JP)**
• **OKUBO, Tetsuo**
**Kanonji-shi**
**Kagawa 769-1602 (JP)**
• **CHEN, Huanhuan**
**Kanonji-shi**
**Kagawa 769-1602 (JP)**

(74) Representative: **Dolleymores**
**9 Rickmansworth Road**
**Watford, Hertfordshire WD18 0JU (GB)**

(56) References cited:
**EP-A1- 1 219 274          JP-A- 2000 210 333**
**JP-A- 2000 354 607          JP-A- 2005 218 876**
**JP-A- 2007 275 322          JP-A- 2015 104 543**
**US-A- 5 672 166          US-A- 6 120 486**

**Description**

[TECHNICAL FIELD]

The present invention relates to a disposable diaper.

[BACKGROUND ART]

Patent Literature 1 discloses a disposable diaper having an absorbent core, an edge portion in which an absorbent core is not present at an outer side of the absorbent core in a width direction, and a three-dimensional gather. The three-dimensional gather of Patent Literature 1 is fixed to both sides of the absorbent core in the width direction, and rises to the wearer side with the vicinity of the edge portion as a base point.

[CITATION LIST]

[PATENT LITERATURE]

Patent Literature 1: JP 2014-180346 A

[SUMMARY OF INVENTION]

[TECHNICAL PROBLEM]

The maximum height in the state where the three-dimensional gather rises is the distance between the fixed part of the three-dimensional gather and the inner edge of the three-dimensional gather. Since the fixed part of the three-dimensional gather is positioned outside the absorbent core in the width direction, the distance between the fixed part of the three-dimensional gather and the inner edge of the three-dimensional gather becomes long and the height of the three-dimensional gather becomes high, as compared with the configuration in which the fixed part is positioned on the absorbent core.

In the crotch region applied to the wearer's crotch, if the height of the three-dimensional gather is too high, the wearer's legs are caught by the three-dimensional gather at the time of wearing, thus deteriorating the operability at the time of wearing.

In addition, in order to make it difficult for the legs of the wearer to be caught on wearing, the distance between the fixed part of the three-dimensional gather and the inner edge of the three-dimensional gather may be designed to be short so as to lower the height of the entire three-dimensional gather. However, if the overall height of the three-dimensional gather is lowered, the volume of the accommodation space for accommodating excrement becomes small and excrement may leak.

The present invention has been made in view of the above problems, and it is an object of the present invention to provide a disposable diaper capable of securing an accommodation space for accommodating excrement and suppressing deterioration in operability at the time of wearing.

[SOLUTION TO PROBLEM]

In summary, to solve the above problem, the disposable diaper (disposable diaper 1) according to the present disclosure is a pants-type disposable diaper, which has a longitudinal direction (longitudinal direction L), a width direction (width direction W) perpendicular to the longitudinal direction, and a thickness direction (thickness direction T), the disposable diaper including: a front side region (front side region 3) positioned at a front side of a center of the disposable diaper in the longitudinal direction, and a rear side region (rear side region 2) positioned at a rear side of the center of the disposable diaper in the longitudinal direction; an absorber (absorber 11) arranged to straddle the front side region and the rear side region; and a pair of three-dimensional gathers (three-dimensional gather 15) having a sheet member (side sheet 14) and an elastic member (three-dimensional gather elastic member 155) and arranged on both outer sides in the width direction with respect to the center of the disposable diaper in the width direction (center 1WC of the disposable diaper in the width direction), wherein outer edges of the front side region and the outer edge of the rear side region are bonded, the three-dimensional gather includes a base end portion incapable of being upright, and an upright portion (upright portion 154) shrinkable in the longitudinal direction by the elastic member and capable of being upright on the skin side in the thickness direction with the base end portion as a base point, the base end portion includes: a first base end portion (first base end portion 151) arranged at each of the front side region and the rear side region at both outer edges of the upright portion in the longitudinal direction; and a second base end portion (second base end portion 152) arranged at the outer edge of the upright portion in the width direction, the upright portion includes a bonded portion (bonded portion 153) in which the sheet members are bonded to each other and a length of the upright portion in the width direction is narrowed, the bonded portion extends over the center of the disposable diaper in the longitudinal direction and is spaced from each of the first base end portions, and a distance (length L4 of the bonded portion in the longitudinal direction in the rear side region) between the center of the disposable diaper in the longitudinal direction and the rear end edge of the bonded portion is longer than a distance (length L5 of the bonded portion in the longitudinal direction in the front side region) between the center of the disposable diaper in the longitudinal direction and the front end edge of the bonded portion.

[ADVANTAGEOUS EFFECTS OF INVENTION]

According to the present disclosure, it is possible to provide a disposable diaper capable of securing an accommodation space for accommodating excrement and suppressing deterioration in operability at the time of wearing.

[BRIEF DESCRIPTION OF DRAWINGS]

Fig. 1 is a perspective image view of a disposable diaper according to an embodiment.
Fig. 2 is a plan view of a disposable diaper in a deployed state.
Fig. 3 is a plan view of a three-dimensional gather.
Fig. 4 is a cross-sectional view along a width direction of a disposable diaper. Fig. 4(a) is a cross-sectional view taken along line A-A in Fig. 2, Fig. 4(b) is a cross-sectional view taken along line B-B in Fig. 2, and Fig. 4(c) is a cross-sectional view taken along line C-C in Fig. 2.
Fig. 5 is a cross-sectional view along a longitudinal direction of a disposable diaper in a state of being worn by a wearer. Fig. 5(a) is a disposable diaper according to a comparative example, and Fig. 5(b) is a disposable diaper according to an embodiment.
Fig. 6 is a cross-sectional view taken along a width direction of an upright portion of a three-dimensional gather. Fig. 6(a) is a disposable diaper according to a comparative example, and Fig. 6(b) is a disposable diaper according to an embodiment.
Fig. 7 is a diagram schematically illustrating a cross-section at a center of a disposable diaper in a longitudinal direction. Fig. 7(a) is a state before wearing, and Fig. 7(b) is a state after wearing.

[DESCRIPTION OF EMBODIMENTS]

At least the following matters will become apparent from the description of this specification and the accompanying drawings.
A pants-type disposable diaper, which has a longitudinal direction, a width direction perpendicular to the longitudinal direction, and a thickness direction, the disposable diaper including:

a front side region positioned at a front side of the center of the disposable diaper in the longitudinal direction, and a rear side region positioned at a rear side of the center of the disposable diaper in the longitudinal direction;
an absorber arranged to straddle the front side region and the rear side region; and
a pair of three-dimensional gathers having a sheet member and an elastic member and
arranged on both outer sides in the width direction with respect to the center of the disposable diaper in the width direction,
wherein the outer edge of the front side region and the outer edge of the rear side region are bonded,
the three-dimensional gather includes a base end portion incapable of being upright, and an upright portion shrinkable in the longitudinal direction by the elastic member and capable of being upright on the skin side in the thickness direction with the base end portion as a base point,
the base end portion includes:

a first base end portion arranged at each of the front side region and the rear side region at both outer edges of the upright portion in the longitudinal direction; and
a second base end portion arranged at the outer edge of the upright portion in the width direction,
the upright portion has a bonded portion in which the sheet members are bonded to each other and the length in the width direction of the upright portion is narrowed,
the bonded portion extends over the center of the disposable diaper in the longitudinal direction and is spaced from each of the first base end portions, and
the distance between the center of the disposable diaper in the longitudinal direction and the rear end edge of the bonded portion is longer than the distance between the center of the disposable diaper in the longitudinal direction and the front end edge of the bonded portion.

According to such a disposable diaper, the upright portion of the three-dimensional gather rises on the wearer's side with the first base end portion and the second base end portion as the base end portion. In the cross-section along the width direction, the upright portion of the three-dimensional gather rises from the second base end portion as a starting point. The height when the three-dimensional gather rises is the distance between the second base end portion and the inner edge of the three-dimensional gather. In addition, in the cross-section along the longitudinal direction, the upright portion of the three-dimensional gather rises from the first base end portion as a starting point. When comparing the upright portion in the vicinity of the first base end portion with the upright portion in the center of the disposable diaper in the longitudinal direction, the upright portion in the vicinity of the first base end portion is relatively close to the first base end portion and pulled by the first base end portion, and thus the height is easily suppressed. On the other hand, the upright portion of the center of the disposable diaper in the longitudinal direction is pulled upwardly through the first base end portion in a worn state, so that it tends to be high. By arranging the bonded portion across the center of the disposable diaper in the longitudinal direction, the distance between the second base end portion and the inner edge of the three-dimensional gather becomes shorter at the center of the disposable diaper in the longitudinal direction, and the height of the three-dimensional gather can be suppressed. Since the height of the three-dimensional gather of the center of the disposable diaper in the longitudinal direction is relatively low, it is difficult for the legs to be caught during wearing, and it is possible to suppress deterioration in operability at the time of wearing.

The distance between the center of the disposable diaper in the longitudinal direction and the rear end edge of the bonded portion is longer than the distance between the center of the disposable diaper in the longitudinal direction and the front end edge of the bonded portion. Therefore, it is possible to effectively suppress the height of the three-dimensional gather at a rear side of the center of the disposable diaper in the longitudinal direction. In general, the shape of the leg surrounding of the wearer more swells in the rear side than the front side. However, since the height of the three-dimensional gather is effectively suppressed at the rear side of the center of the disposable diaper in the longitudinal direction, when the disposable diaper is raised at the time of wearing, it is difficult for the back side of the leg to be caught and it is possible to suppress the three-dimensional gather from rolling over outward in the width direction. Therefore, when the disposable diaper is easily pulled up at the time of wearing, deterioration of operability at the time of wearing can be suppressed. In addition, particularly in the rear side region, it is possible to suppress the collapse of the three-dimensional gather toward the outside in the width direction, and the three-dimensional gather is arranged so as to cover the swelling of the buttocks, thereby suppressing leakage of excrement.

In addition, the bonded portion and each first base end portion are spaced apart. The distance between the second base end portion and the inner edge of the three-dimensional gather is relatively long in the region between the bonded portion and the first base end portion. In the region where the bonded portion is not provided, the accommodation space by the three-dimensional gather can be made large and the leakage of excrement can be suppressed.

In such a disposable diaper, the distance between the first base end portion of the rear side region and the bonded portion is preferably shorter than the distance between the first base end portion of the front side region and the bonded portion.

According to such a disposable diaper, the bonded portion in the rear side region is closer to the first base end portion than the bonded portion of the front side region. Compared to the inner edge of the three-dimensional gather in the front side region, the inner edge of the three-dimensional gather in the rear side region tends to be pulled by the second base end portion and the first base end portion and the elastic member tends to be stretched. Therefore, the inner edge of the three-dimensional gather in the rear side region is easily pulled by the first base end portion and easily arranged inside the second base end portion in the width direction. In addition, since the elastic member of the three-dimensional gather in the rear side region tends to be stretched, it is easy to adopt a configuration in which the three-dimensional gather rises and the topsheet is covered while keeping space.

In addition, the three-dimensional gather in the rear side region becomes difficult to deploy so that the inner edge of the three-dimensional gather collapses outward in the width direction at the time of wearing. It is easier to provide a stool pocket by the three-dimensional gather, and leakage of excrement can be suppressed.

In such a disposable diaper, the distance between the first base end portion in the front side region and the front end edge of the bonded portion is longer than the distance between the center of the disposable diaper in the longitudinal direction and the front end edge of the bonded portion.

According to such a disposable diaper, since the region where the bonded portion is not provided is relatively wide at the front side of the center of the disposable diaper in the longitudinal direction, it is possible to secure the height of the three-dimensional gather. Excrement discharged to the front side region is mainly urine and has higher fluidity than feces. If it cannot be absorbed immediately at the time of massive urination, a relatively high three-dimensional gather functions as a breakwater. In addition, since the height of the three-dimensional gather at the front side of the center of the disposable diaper in the longitudinal direction is secured, the area covering the absorber that absorbed the urine can be widened and the feeling of fit can be improved.

In such a disposable diaper, it is preferable that the distance between the first base end portion in the rear side region and the rear end edge of the bonded portion is shorter than the total length of the length of the first base end portion in the rear side region in the longitudinal direction and the length of the bonded portion in the rear side region in the longitudinal direction and is longer than half the total length.

According to such a disposable diaper, since the distance between the first base end portion in the rear side region and the rear end edge of the bonded portion is made shorter than the total length, the region where the length of the upright portion in the width direction in the rear side region is relatively long is short, and it is possible to suppress the height of the three-dimensional gather. In addition, since the distance between the first base end portion in the rear side region and the rear end edge of the bonded portion is made longer than half the total length, it is possible to widen the accommodation space by the three-dimensional gather at the portion corresponding to the dorsal side of the wearer. In the rear side region, it is easier to maintain the form in which the three-dimensional gather covers the absorber while maintaining the space for accommodating excrement.

Such a disposable diaper includes

a leg gather arranged on the outer side of the three-dimensional gather in the width direction,
the leg gather includes a plurality of leg elastic members arranged at a distance in the width direction, and
it is desirable that the elongation rate of the outer leg elastic member positioned on the outermost side in the width direction is lower than the elongation rate of the leg elastic member positioned at an inner side of the outer leg elastic member in the width direction.

According to such a disposable diaper, since the elongation rate of the outer leg elastic member is relatively low, the height of the outer edge of the leg gather does not become too high. The opening can be formed wider toward the outside in the width direction, thereby making it easier to pass the legs at the time of wearing. In addition, since the plurality of leg elastic members are arranged at a distance in the width direction, the leg gathers have a certain length in the width direction and become planar. Since the leg gathers are planar, the legs can be passed along the leg gathers and the legs can be passed smoothly.

In such a disposable diaper,

the elongation rate of the inner leg elastic member positioned on the innermost side in the width direction is higher than the elongation rate of the leg elastic member positioned at an outer side of the inner leg elastic member in the width direction, and
the length of the leg gather in the width direction at the center of the disposable diaper in the longitudinal direction is preferably longer than the distance between the second base end portion in the center of the disposable diaper in the longitudinal direction and the inner edge of the three-dimensional gather.

According to such a disposable diaper, since the elongation rate of the inner leg elastic member is high, the leg gather rises to the wearer side with the inner leg elastic member as the apex. In addition, since the elongation rate of the leg elastic member positioned on the outer side of the inner leg elastic member in the width direction is relatively low, it is possible to expand the wall by the leg gather from the inner leg elastic member toward the outside in the width direction. Since the wall with the inner leg elastic member as the apex can be formed on the outside in the width direction of the three-dimensional gather, side leakage of excrement can be prevented.

In addition, at the center of the disposable diaper in the longitudinal direction, since the length of the leg gather in the width direction is longer than the distance between the second base end portion and the inner edge of the three-dimensional gather, it is possible to form the wall by leg gather even when the three-dimensional gather falls to the outside in the width direction. Therefore, leakage of excrement can be suppressed while maintaining operability at the time of wearing.

=== Regarding Absorbent Article of the Present Embodiment ===

Next, an embodiment of a disposable diaper 1 according to the present invention will be described with reference to the drawings. In the following description of the drawings, the same or similar reference numerals are assigned to the same or similar parts. However, it should be noted that the drawings are schematic and ratios of dimensions and the like may be different from actual ones. Therefore, concrete dimensions and the like should be determined with reference to the following explanation. In addition, the drawings may include portions having different dimensional relationships or ratios.

(1) Overall Schematic Configuration of Disposable Diaper

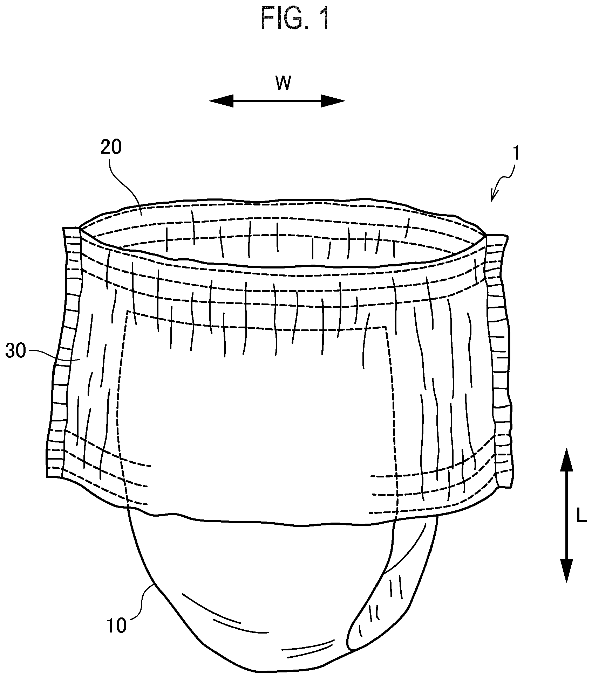

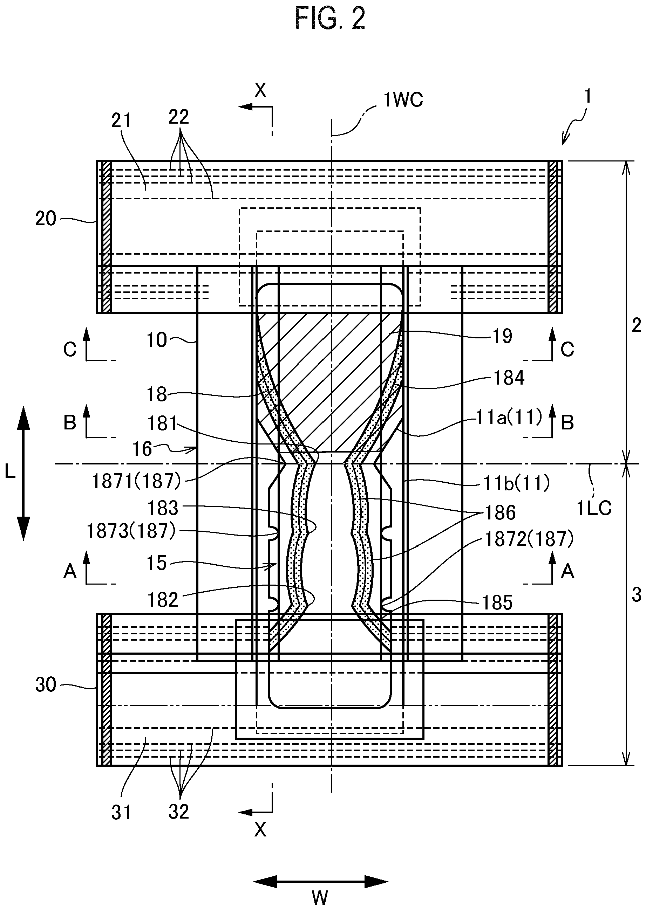

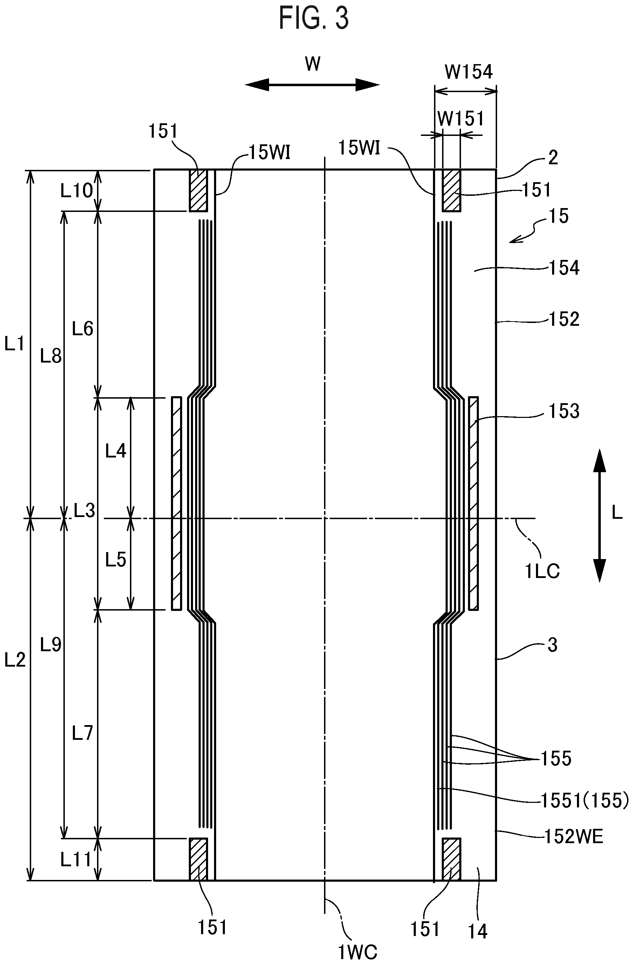

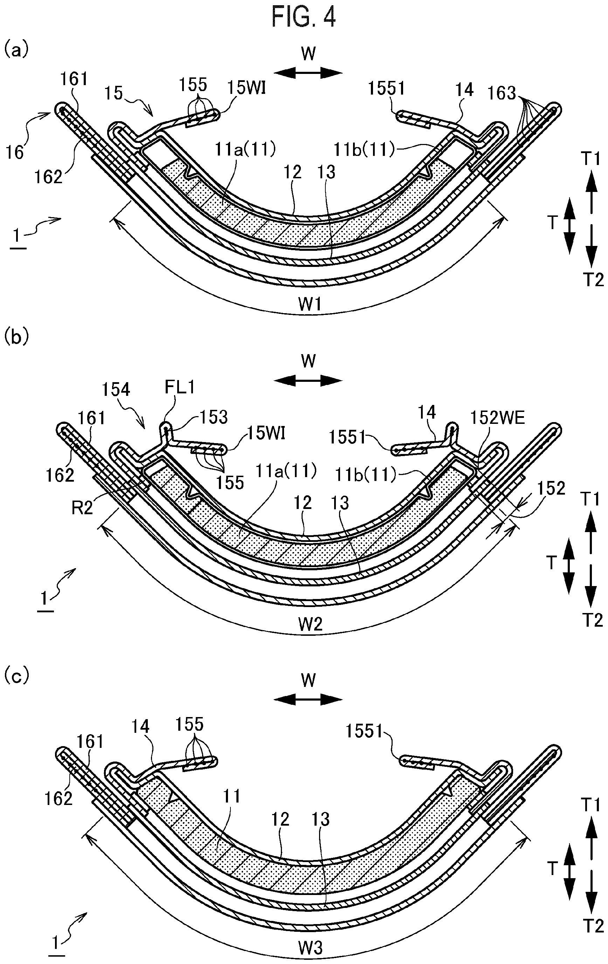

Fig. 1 is a perspective image view of a disposable diaper according to an embodiment. Fig. 2 is a plan view of the disposable diaper in a deployed state. Fig. 3 is a plan view of a three-dimensional gather. Fig. 4 is a cross-sectional view along the width direction of the disposable diaper. The plan view shown in Fig. 2 and the plan view shown in Fig. 3 are diagrams in a stretched state in which an elastic member such as a three-dimensional gather elastic member 155 is stretched, up to a state where wrinkles of a topsheet 12, a side sheet 14, and a three-dimensional gather 15 constituting the disposable diaper are not formed.

The disposable diaper 1 has a longitudinal direction L, a width direction W perpendicular to the longitudinal direction L, and a thickness direction T. The longitudinal direction L is a direction connecting a front side exterior body 30 arranged on the ventral side of the wearer and a rear side exterior body 20 arranged on the dorsal side of the wearer. The thickness direction T is a direction connecting a skin side T1, which is a direction contacting the wearer, and a non-skin side T2 opposite thereto.

The disposable diaper 1 includes a rear side region 2 positioned at a rear side of the center 1LC of the disposable diaper in the longitudinal direction, and a front side region 3 positioned at a front side of the center 1LC of the disposable diaper in the longitudinal direction. The disposable diaper 1 is a so-called three-piece type and has three parts. The disposable diaper 1 includes an absorbent main body, as a first part, which is applied to the wearer's crotch and absorbs excrement, a rear side exterior body 20, as a second part, which covers the dorsal side portion of the wearer, and a front side exterior body 30, as a third part, which covers the ventral side portion of the wearer.

As illustrated in Fig. 4, the absorbent main body 10 includes at least an absorber 11, a topsheet 12, a back sheet 13, and a side sheet 14. The absorber 11 includes an absorbent core 11a appropriately constituted by using known members and materials such as a ground pulp and a super-absorbent polymer. The absorbent core 11a may be wrapped by a core wrap 11b. The structure of the absorber 11 will be described in detail later.

A liquid-permeable topsheet 12 is provided on the front surface side (skin side) of the absorber 11. The topsheet 12 covers the skin surface side of the absorber 11. The outer edge of the topsheet 12 extends in the width direction at an outer side of the absorber 11. A liquid-impermeable back sheet 13 is provided on the non-skin side of the absorber 11. The side sheets 14 are provided on the outer edge (the outer end in the width direction) of the absorber 11, respectively. The side sheet 14 covers the outer edge of the absorber 11 and extends in the width direction at the outer side of the absorber 11. The inner edge of the side sheet is arranged on the absorber 11. The side sheet 14 extending in the width direction at the outer side of the absorber 11 is folded back to the non-skin side at the base point positioned at the outer side of the outer edge of the absorber 11 in the width direction. A part of the side sheet 14 folded back to the non-skin side is arranged on the non-skin side of the absorber 11.

The absorbent main body 10 includes a three-dimensional gather 15 and a leg gather 16. The three-dimensional gather 15 includes a side sheet 14 as a sheet member and a three-dimensional gather elastic member 155 as an elastic member. The three-dimensional gathers 15 are arranged in pairs on both outer sides in the width direction of the center 1WC of the disposable diaper in the width direction. The three-dimensional gather may be made of a sheet member different from the side sheet 14. The three-dimensional gather 15 will be described in detail later.

The leg gathers 16 are arranged in pairs on both outer sides of the three-dimensional gathers 15 in the width direction. The leg gather 16 includes a nonwoven fabric sheet 161, a film sheet 162, and a leg gather elastic member 163. The nonwoven fabric sheet 161 is folded inward in the width direction with the outer edge of the absorbent main body 10 as a base point and is stacked in two layers. The film sheet 162 is arranged between the nonwoven fabric sheets 161. The leg gather elastic member 163 is arranged between the nonwoven fabric sheet positioned on the non-skin side of the two nonwoven fabric sheets and the film sheet 162. The leg gather elastic member 163 is constituted by a thread rubber, and six leg gather elastic members 163 are arranged for each of the left and right leg gathers 16. The leg gather elastic members 163 can be stretched in the longitudinal direction and are arranged at intervals in the width direction. The leg gather will be described in detail later.

As illustrated in Fig. 2, the rear side exterior body 20 includes at least a rear side sheet 21 and a rear side elastic member 22. The rear side sheet 21 is made of a nonwoven fabric and is stacked in at least two layers. The rear side elastic member 22 is arranged between the rear side sheets 21 and is stretchable in the width direction. As illustrated in Fig. 2, the front side exterior body 30 includes at least a front side sheet 31 and a front side elastic member 32. The front side sheet 31 is made of a nonwoven fabric and is stacked in at least two layers. The front side elastic member 32 is arranged between the front side sheets 31 and is stretchable in the width direction.

In the deployed state illustrated in Fig. 2, the rear side exterior body 20 and the front side exterior body 30 are juxtaposed in parallel at intervals in the longitudinal direction, and the absorbent main body 10 is stretched between the rear side exterior body 20 and the front side exterior body 30. The front end edge of the absorbent main body is fixed to the front side exterior body 30, and the rear end edge of the absorbent main body is fixed to the rear side exterior body 20. The disposable diaper is folded in half from the state illustrated in Fig. 2 with the center 1LC of the disposable diaper 1 in the longitudinal direction as a folding base point. In the state of being folded in half, the disposable diaper is formed in the pants type illustrated in Fig. 1 by bonding the outer edge of the rear side exterior body 20 which is the outer edge of the rear side region and the outer side edge of the front side exterior body 30 which is the outer side of the front side region.

(2) Three-dimensional gather

Next, the three-dimensional gather 15 will be described in detail. Fig. 3 is a plan view schematically illustrating the plane of the three-dimensional gather 15. The three-dimensional gather 15 is a gather which can be upright on the wearer's side at the time of wearing. The pair of three-dimensional gathers 15 is line-symmetric with respect to the center 1WC of the disposable diaper in the width direction as the axis of symmetry. Since the configurations of both the three-dimensional gathers 15 are the same, one three-dimensional gather 15 will be described below. The three-dimensional gather 15 forms a wall rising to the skin side at the outer edge of the absorber 11, prevents lateral leakage of excrement, covers the inner side in the width direction from the outer edge of the absorber 11, and forms a pocket for accommodating excrement with the absorber 11.

The three-dimensional gather 15 includes a base end portion which cannot be upright, and an upright portion 154 that contracts in the longitudinal direction by the three-dimensional gather elastic member 155 and can be upright on the skin side in the thickness direction with the base end portion as a fulcrum. The front end edge of the three-dimensional gather is the front end edge of the side sheet, and the rear end edge of the three-dimensional gather is the rear end edge of the side sheet. The base end portion includes a first base end portion 151 and a second base end portion 152. Fig. 4(a) is a cross-sectional view taken along line A-A of Fig. 2 in the width direction of the disposable diaper. Fig. 4(c) is a cross-sectional view taken along line B-B of Fig. 2 in the width direction of the disposable diaper. Fig. 4(c) is a cross-sectional view taken along line C-C of Fig. 2 in the width direction of the disposable diaper. Fig. 4(a) is a cross-sectional view of the front side region, and Figs. 4(b) and 4(c) are cross-sectional views of the rear side region.

The first base end portion 151 is arranged at both end edges of the upright portion 154 in the longitudinal direction. The pair of first base end portions 151 spaced apart in the longitudinal direction is arranged with the upright portion 154 interposed therebetween in the longitudinal direction. One first base end portion 151 is arranged on the rear side exterior body 20, and the other first base end portion 151 is arranged on the front side exterior body 30. The first base end portion 151 of the present embodiment is a portion where the side sheet 14 and the topsheet 12 are bonded on the skin side of the absorber. The inner end edge (the inner end in the longitudinal direction) of each first base end portion 151 is a portion that becomes the base point of the rising of the upright portion 154.

The first base end portion 151 is positioned inside the second base end portion 152 in the width direction. The inner edge 15WI of the three-dimensional gather 15 is pulled by the first base end portion 151 and tends to collapse inward in the width direction. The width 151W of the first base end portion 151 may be shorter than the entire width 154W of the upright portion, or may be the same as the entire width 154W of the upright portion. Since the width 151W of the first base end portion 151 is the same as the entire width 154W of the upright portion, the inner edge 15WI of the three-dimensional gather 15 tends to collapse more inward in the width direction.

The second base end portion 152 is a portion configured to be incapable of being upright in a region extending outwardly in the width direction from the upright portion 154, and a portion arranged on the non-skin side of the absorber 11. The second base end portion 152 is arranged adjacent to the outer edge of the upright portion 154. At the second base end portion 152, the side sheet 14 and the absorber 11 are bonded through a bonded portion R2. The outer edge 152WE of the second base end portion 152 is a position corresponding to the outer edge of the absorber 11 and is a portion to be the rising base point of the upright portion 154.

The side sheet 14 and the topsheet 12 may be bonded through the bonded portion R2 in a region outside the upright portion 154 in the width direction. In the configuration in which the bonded portion is positioned on the skin side of the absorber, the bonded portion constitutes the second base end portion. The second base end portion 152 may be positioned on the outer side of the absorber 11 in the width direction, or may overlap the absorber 11.

In addition, the side sheet 14 extends at the outer side of the outer edge of the absorber in the width direction, and is stacked in a portion extending outward in the width direction. In this manner, since the side sheet is folded at the outer side of the outer edge of the absorber in the width direction, the dimension of the three-dimensional gather in the width direction can be increased. In addition, since the side sheet is folded on the outer side of the outer edge of the absorber in the width direction, the area covering the skin side of the absorber can be reduced by the side sheet and it is easy to maintain absorption performance.

The upright portion 154 is positioned at the inner side of the first base end portion 151 in the longitudinal direction and at the inner side of the second base end portion 152 in the width direction. The upright portion 154 includes the inner edge (the inner edge of the side sheet 14) 15WI of the three-dimensional gather 15. The upright portion 154 is a portion where the side sheet 14 is not fixed to the topsheet or the like and is a portion configured to be upright on the skin side. The length of the upright portion 154 in the width direction is the distance between the second base end portion 152 and the inner edge 15WI of the three-dimensional gather 15, and is the maximum height at which a three-dimensional gather can rise. The distance between the second base end portion 152 and the inner edge 15WI of the three-dimensional gather 15 is the distance between the outer edge of the second base end portion 152 and the inner edge 15WI of the three-dimensional gather 15 in a state in which the three-dimensional gather is developed outward in the width direction with the outer edge of the second base end portion 152 as a base point and wrinkles of the three-dimensional gather are extended to a state where wrinkles are not visible.

The upright portion 154 includes a bonded portion 153 to which side sheets as a sheet member are bonded. The bonded portion 153 is a portion that bonds the side sheets 14 to each other and narrows the length of the upright portion 154 in the width direction. The length of the upright portion 154 in the width direction is the length of the upright portion 154 along the width direction W in the extended state illustrated in Fig. 3 (the distance between the second base end portion 152 and the inner edge of the three-dimensional gather 15), and is the rising height of the upright portion 154 in state in which the upright portion 154 contracts and rises to the wearer side as illustrated in Fig. 4.

The bonded portion 153 is a portion bonded by, for example, an adhesive, thermal welding, or ultrasonic welding. Fig. 4(b) is a cross-sectional view of the region where the bonded portion 153 is provided. The bonded portion 153 is a portion that is folded with the folding line FL1 extending in the longitudinal direction as a base point such that the side sheets 14 overlapping in two layers are bonded together. As illustrated in Fig. 4(b), the bonded portion 153 is arranged outside the space between the three-dimensional gather 15 and the absorber 11. The distance between the second base end portion 152 and the inner edge of the three-dimensional gather 15 in the region where the bonded portion 153 is provided is shorter than the distance between the second base end portion 152 and the inner edge of the three-dimensional gather 15 in the region where the bonded portion 153 is not provided. Therefore, in the region where the bonded portion 153 is provided, the height of the three-dimensional gather can be suppressed. The upright portion in the cross-section illustrated in Fig. 4(b) is in a state of being lying more than the upright portion in the cross-section illustrated in Fig. 4(a) and the upright portion in the cross-section illustrated in Fig. 4(c), and the height is suppressed.

In addition, since the distance between the second base end portion 152 and the inner edge of the three-dimensional gather 15 is relatively short, the tension between the three-dimensional gather elastic member 155 and the second base end portion 152 and the tension between the three-dimensional gather elastic member 155 and the first base end portion 151 are likely to be maintained, and it becomes difficult to deploy so that the inner edge of the three-dimensional gather 15 collapses outward in the width direction at the time of wearing. Therefore, it becomes easier to provide a stool pocket by the three-dimensional gather 15, and leakage of excrement can be suppressed.

The bonded portion 153 is arranged at least in the rear side region 2. Since the bonded portion 153 is arranged in the rear side region 2, it is easy to form a pocket for accommodating feces in the rear side region 2. It becomes difficult for the feces to touch the wearer's skin, and the feeling of fit can be improved. The bonded portion 153 of the present embodiment straddles the center of the disposable diaper in the longitudinal direction. That is, the bonded portion 153 is arranged in both the front side region 3 and the rear side region 2. Since the bonded portion 153 is arranged in both the front side region 3 and the rear side region 2, it is easy to form pockets for accommodating excrement in both the front side region 3 and the rear side region 2.

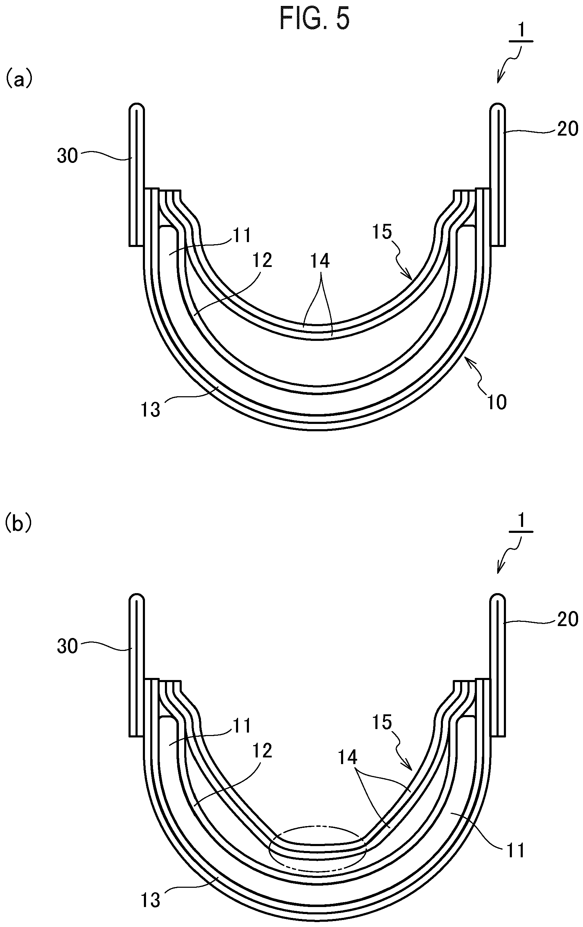

The height of the upright portion 154 positioned at the center of the disposable diaper in the longitudinal direction is suppressed, and it is possible to obtain the effect that it is difficult for the legs to be caught at the time of wearing. Fig. 5 is a schematic cross-sectional view of a mounted state in a cross-section taken along line X-X illustrated in Fig. 2. Fig. 5(a) illustrates a disposable diaper according to a comparative example without a bonded portion, and Fig. 5(b) illustrates a disposable diaper according to an embodiment with a bonded portion. In Fig. 5(b), a portion surrounded by a chain double-dashed line is a portion whose height is suppressed by the bonded portion. By placing the bonded portion across the center of the disposable diaper in the longitudinal direction, the height of the three-dimensional gather can be suppressed at the center of the disposable diaper in the longitudinal direction.

In the cross-section along the longitudinal direction, the upright portion 154 of the three-dimensional gather rises from the first base end portion 151 as a starting point. When comparing the upright portion 154 in the vicinity of the first base end portion with the upright portion 154 in the center of the disposable diaper in the longitudinal direction, the upright portion 154 in the vicinity of the first base end portion is relatively close to the first base end portion 151 and pulled by the first base end portion 151, and thus the height is easily suppressed. On the other hand, the upright portion 154 of the center of the disposable diaper in the longitudinal direction is pulled upwardly through the first base end portion 151 in a worn state, so that it tends to be high. By arranging the bonded portion across the center of the disposable diaper in the longitudinal direction, the distance between the second base end portion 152 and the inner edge 15WI of the three-dimensional gather 15 becomes shorter at the center of the disposable diaper in the longitudinal direction, and the height of the three-dimensional gather can be suppressed. Since the height of the three-dimensional gather of the center of the disposable diaper in the longitudinal direction is relatively low, it is difficult for the legs to be caught during wearing, and it is possible to suppress deterioration in operability at the time of wearing.

In addition, since the height of the three-dimensional gather at the center in the longitudinal direction of the disposable diaper is relatively low, it is possible to prevent the inner edge 15WI of the three-dimensional gather 15 from excessively entering the inside in the width direction and it is easy to arrange the inner edge 15WI of the three-dimensional gather 15 so as to cover the crotch of the wearer. In addition, since the bonded portion 153 is arranged across the center of the disposable diaper in the longitudinal direction, the upright portion 154 positioned at the center of the disposable diaper in the longitudinal direction has a shorter distance to the second base end portion 152 and is pulled outward in the width direction. Therefore, the tension of the three-dimensional gather elastic member 155 positioned at the center of the disposable diaper in the longitudinal direction tends to maintain a high state. The upright portion 154 positioned at the center of the disposable diaper in the longitudinal direction tends to be pulled by the first base end portion 151 and tends to maintain a state in which it falls inward in the width direction. In addition, the upright portion 154 positioned at the center of the disposable diaper in the longitudinal direction is pulled by the second base end portion 152 and pulled outward in the width direction. Therefore, it is easy to maintain the state in which the three-dimensional gather 15 rises on the side of the wearer, and it is possible to provide a wide space between the left and right three-dimensional gathers 15. The upright portion of the three-dimensional gather is pulled toward the second base end portion side and pulled toward the first base end portion side and becomes a state slightly falling inward in the width direction. Therefore, the space for accommodating feces is formed between the three-dimensional gather and the absorber, and feces can be prevented from touching the skin directly by the three-dimensional gather positioned between the feces and the skin.

The length L1 of the three-dimensional gather in the longitudinal direction in the rear side region 2 is shorter than the length L2 of the three-dimensional gather 15 in the longitudinal direction in the front side region 3. According to such a configuration, the three-dimensional gather of the rear side region 2 tends to exhibit the effect of falling inward in the width direction by the first base end portion 151 as compared with the front side region 3. Therefore, it is possible to effectively suppress the height of the three-dimensional gather in the rear side region. The upright portion in the cross-section illustrated in Fig. 4(c) is in a state of being lying more than the upright portion in the cross-section illustrated in Fig. 4(a), and the height is suppressed.

The front end edge of the bonded portion 153 is spaced apart from the first base end portion 151 positioned in the front side region 3 and is positioned at a rear side of the first base end portion 151 positioned in the front side region 3. The distance between the second base end portion 152 and the inner edge of the three-dimensional gather 15 is relatively long between the first base end portion 151 positioned in the front side region 3 and the bonded portion 153. In the region where the bonded portion 153 is not provided, it is possible to form a large pocket by the three-dimensional gather 15, and it is possible to secure a wide accommodation space for the pocket. The rear end edge of the bonded portion 153 is spaced apart from the first base end portion 151 positioned in the rear side region 2 and is positioned at a front side of the first base end portion 151 positioned in the rear side region 2. The distance between the second base end portion 152 and the inner edge of the three-dimensional gather 15 is relatively long between the first base end portion 151 positioned in the rear side region 2 and the bonded portion 153. In the region where the bonded portion 153 is not provided, it is possible to form a large pocket by the three-dimensional gather 15, and it is possible to secure a wide accommodation space for the pocket.

The length L3 of the bonded portion 153 in the longitudinal direction is longer than the distance L6 between the bonded portion 153 and the first base end portion 151 positioned in the rear side region 2, and is shorter than the distance L7 between the bonded portion 153 and the first base end portion 151 positioned in the front side region 3.

The length L4 of the bonded portion 153 of the rear side region 2 in the longitudinal direction L is longer than the length L5 of the bonded portion 153 of the front side region 3 in the longitudinal direction L. The length of the bonded portion 153 positioned in the rear side region 2 is the distance between the rear end edge of the bonded portion 153 and the center of the disposable diaper in the longitudinal direction. The length of the bonded portion 153 positioned in the front side region 3 is the distance between the front end edge of the bonded portion 153 and the center of the disposable diaper in the longitudinal direction. Since the length of the bonded portion 153 positioned in the rear side region 2 is longer than the length of the bonded portion 153 positioned in the front side region 3, it is possible to effectively suppress the height of the three-dimensional gather in the rear side region. In general, the shape of the leg surrounding of the wearer more swells in the rear side than the front side. Since the height of the three-dimensional gather is effectively suppressed at the rear side of the center of the disposable diaper in the longitudinal direction, when the disposable diaper is raised at the time of wearing, it is difficult for the back side of the leg to be caught and it is possible to suppress the three-dimensional gather from rolling over outward in the width direction. Therefore, when the disposable diaper is easily pulled up at the time of wearing, deterioration of operability at the time of wearing can be suppressed. In addition, it is possible to effectively suppress the height of the three-dimensional gather 15 in the rear side region 2 becoming too high, and it is possible to further suppress the collapse of the three-dimensional gather 15 due to excessive rising of the inner edge of the three-dimensional gather 15.

The distance L7 between the first base end portion 151 positioned in the front side region 3 and the front end edge of the bonded portion 153 is longer than the distance L6 between the first base end portion 151 positioned in the rear side region 2 and the rear end edge of the bonded portion 153. In the front side region, since the length of the region where the bonded portion is not provided is relatively long, the height of the three-dimensional gather can be secured. Excrement discharged to the front side region are mainly urine and have high fluidity. If it cannot be absorbed immediately at the time of massive urination, a relatively high three-dimensional gather functions as a breakwater. Therefore, it is possible to suppress leakage of excrement in the front side region. In addition, since the height of the three-dimensional gather at the front side region is secured, the area covering the absorber that absorbed the urine can be widened and the feeling of fit can be improved.

In addition, the bonded portion 153 in the rear side region 2 is closer to the first base end portion 151 than the bonded portion 153 of the front side region 3. Compared to the inner edge 15WI of the three-dimensional gather 15 in the front side region, the inner edge 15WI of the three-dimensional gather 15 in the rear side region tends to be pulled by the second base end portion 152 and the first base end portion 151 and the elastic member tends to be stretched. Therefore, the inner edge 15WI of the three-dimensional gather 15 in the rear side region is easily pulled by the first base end portion 151 and easily arranged inside the second base end portion 152 in the width direction. In addition, the three-dimensional gather in the rear side region becomes difficult to deploy so that the inner edge 15WI of the three-dimensional gather 15 collapses outward in the width direction at the time of wearing. That is, by suppressing the height of the three-dimensional gather 15, the effect that the three-dimensional gather 15 collapses inward in the width direction can be more easily exerted in the rear side region 2. In addition, since the elastic member of the three-dimensional gather in the rear side region tends to be stretched, it is easy to adopt a configuration in which the three-dimensional gather stands up and the topsheet is covered while keeping space. Therefore, it becomes easier to provide a stool pocket by the three-dimensional gather, and leakage of excrement can be suppressed.

The ratio of the length L4 of the bonded portion 153 to the length L8 of the upright portion 154 in the rear side region 2 is higher than the ratio of the length L5 of the bonded portion 153 to the length L9 of the upright portion 154 in the front side region 3. The length L8 of the upright portion 154 in the rear side region 2 is the distance between the first base end portion 151 positioned in the rear side region 2 and the center of the disposable diaper in the longitudinal direction, and is the length of the region which can rise in the rear side region 2. Similarly, the length of the upright portion 154 in the front side region 3 is the distance between the first base end portion 151 positioned in the front side region 3 and the center of the disposable diaper in the longitudinal direction, and is the length of the region that can rise in the front side region 3.

The ratio of the region where the bonded portion 153 in the upright portion 154 of the rear side region 2 is arranged is higher than the ratio of the region where the bonded portion 153 in the upright portion 154 in the front side region 3 is arranged. Therefore, in the rear side region 2, the ratio of the region which is likely to be relatively low is large, and the height of the upright portion 154 is easily suppressed. It is possible to effectively suppress the height of the three-dimensional gather 15 of the rear side region 2 from becoming excessively high, to prevent the legs from being caught at the time of wearing, and to suppress deterioration in operability at the time of wearing. In addition, it is possible to further suppress the collapse of the three-dimensional gather 15 caused by the excessive rising of the inner edge of the three-dimensional gather 15 in the rear side region. Therefore, it is possible to provide a wide space between the left and right three-dimensional gathers 15 in the rear side region.

On the other hand, in the front side region 3, it is possible to secure the height of the three-dimensional gather 15 as compared with the rear side region 2. Excrement discharged to the front side region 3 are mainly urine and have high fluidity. If it cannot be absorbed immediately at the time of massive urination, a relatively high three-dimensional gather functions as a breakwater.

In addition, it is preferable that the ratio of the length of the bonded portion of the three-dimensional gather in the longitudinal direction and the distance to the region without the bonded portion (the distance between the first base end portion and the bonded portion) satisfies the following Formulae 1 and 2. When satisfying Formula 1, it is possible to cover the absorber by the three-dimensional gather while providing a space between the three-dimensional gather and the absorber in the rear side region. Further, when satisfying Expression 2, the three-dimensional gather can easily rise.

(Formula 1)

(the length L10 of the first base end portion 151 positioned in the rear side region in the longitudinal direction + the length L4 of the bonded portion in the longitudinal direction in the rear side region) > (the distance L6 between the first base end portion 151 of the rear side region and the bonded portion) > (the length L10 of the first base end portion 151 positioned in the rear side region in the longitudinal direction + the length L4 of the bonded portion in the longitudinal direction in the rear side region)/2

(Formula 2)

(the distance L7 between the first base end portion 151 in the front side region and the bonded portion) > (the length L11 of the first base end portion 151 positioned in the front side region in the longitudinal direction + the length L5 of the bonded portion in the longitudinal direction in the front side region)

When the distance between the first base end portion 151 in the rear side region and the rear end edge of the bonded portion is made shorter than the total length, the region where the length of the upright portion 154 in the width direction in the rear side region is relatively long is short, and it is possible to suppress the height of the three-dimensional gather. In addition, since the distance between the first base end portion 151 in the rear side region and the rear end edge of the bonded portion is made longer than half the total length, it is possible to widen the accommodation space by the three-dimensional gather at the portion corresponding to the dorsal side of the wearer. In the rear side region, it is easier to maintain the form in which the three-dimensional gather covers the absorber while maintaining the space for accommodating excrement.

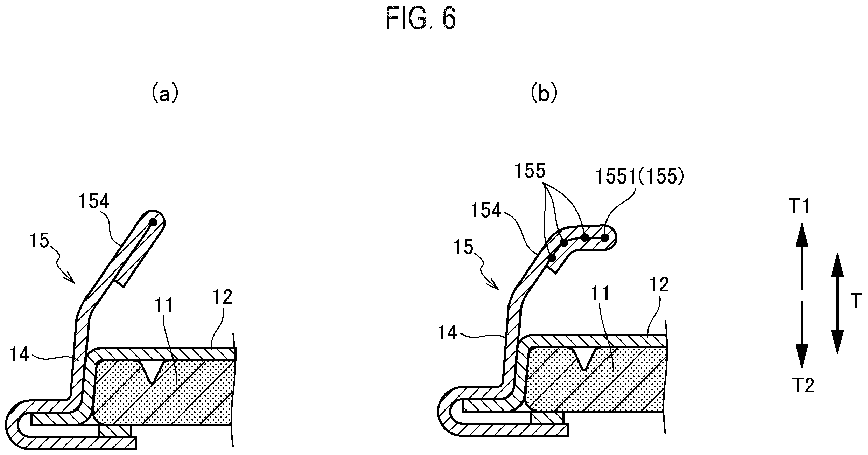

The three-dimensional gather elastic member 155 is made of a thread rubber, and four elastic three-dimensional gather elastic members 155 are arranged for each of the left and right three-dimensional gathers 15. The three-dimensional gather elastic members 155 can be stretched in the longitudinal direction and are arranged at intervals in the width direction. Fig. 6 is a diagram schematically illustrating a cross-section taken along the width direction of the region where the three-dimensional gather elastic member is provided. Fig. 6(a) illustrates a comparative example in which one three-dimensional gather elastic member is used, and Fig. 6(b) illustrates the three-dimensional gather according to the embodiment.

Since a plurality of three-dimensional gather elastic members 155 are arranged at intervals in the width direction, the portion contracted by the three-dimensional gather elastic member 155 has a certain length in the width direction and becomes a planar shape. More specifically, since the plurality of three-dimensional gather elastic members 155 are provided, the entire region where the plurality of three-dimensional gather elastic members 155 are arranged is contracted in the longitudinal direction, the entire region where the plurality of three-dimensional gather elastic members 155 are arranged is pushed up to the wearer side, and the portion contracted by the three-dimensional gather elastic member 155 is formed in a planar shape. Since the portion contracted by the three-dimensional gather elastic member 155 is planar, the length of the feces pocket in the width direction can be easily secured. In addition, since the portion contracted by the three-dimensional gather elastic member 155 is planar, it is possible to reduce the contact with the skin, as compared with the configuration in which one three-dimensional gather elastic member 155 is contracted.

The inner elastic member 1551 positioned on the innermost side of the three-dimensional gather elastic member 155 in the width direction is positioned on the inner edge of the three-dimensional gather 15. The elongation rate of the inner elastic member 1551 is higher than the elongation rate of the three-dimensional gather elastic member 155 positioned at the outer side of the inner elastic member 1551 in the width direction. Compared with other elastic members constituting the three-dimensional gather elastic member 155, the distance between the inner elastic member 1551 and the second base end portion 152 is long, and it is difficult for the inner elastic member 1551 to be pulled by the second base end portion 152. However, since the elongation rate of the inner elastic member 1551 is higher than the elongation rate of the other three-dimensional gather elastic member 155, the deflection of the inner elastic member 1551 is suppressed and the feces pocket is more easily formed.

(3) Leg Gather

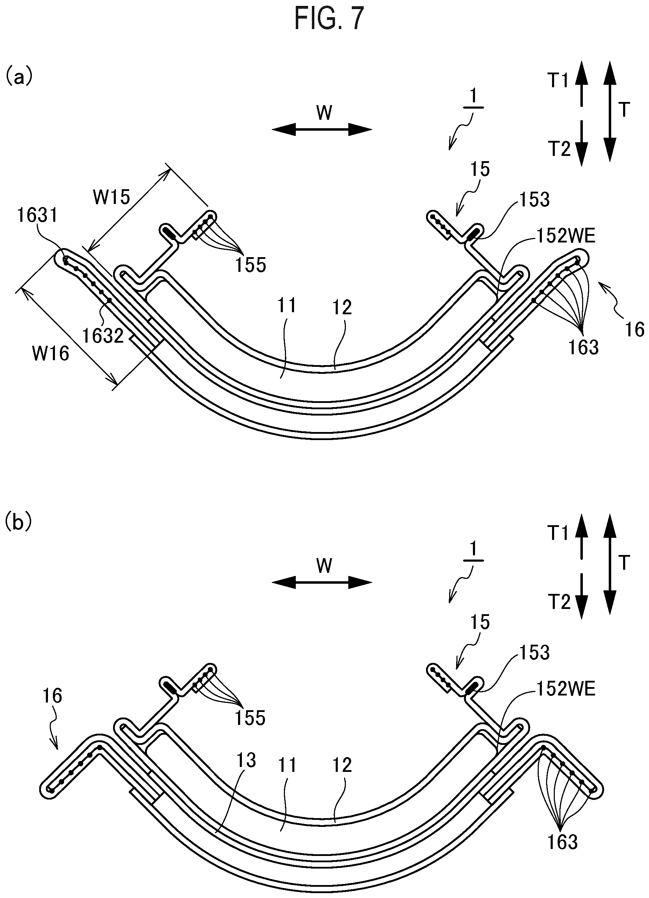

Next, the leg gather will be described. Fig. 7 is a diagram schematically illustrating a cross-section at the center of the disposable diaper in a longitudinal direction. Fig. 7(a) illustrates a state before wearing, and Fig. 7(b) illustrates a state after wearing. In the state before wearing, due to the contraction of the leg gather and the contraction of the three-dimensional gather, the outer edge of the disposable diaper is positioned on the skin side T1 from the center of the disposable diaper in the width direction. The leg gather is arranged on the outer edge of the disposable diaper. Therefore, when the disposable diaper is worn, the leg gathers are arranged closer to the wearer's side, and the wearer's legs can be smoothly inserted.

A plurality of leg elastic members are arranged at intervals in the width direction. According to such a configuration, the leg gather has a certain length in the width direction and becomes a planar shape. Since the leg gathers are planar, the legs can be passed along the leg gathers and the legs can be passed smoothly.

The elongation rate of the outer leg elastic member 1631 positioned at the outermost side in the width direction among the plurality of leg elastic members is lower than the elongation rate of the other leg elastic members positioned at the inner side of the outer leg elastic member 1631 in the width direction. Since the elongation rate of the outer leg elastic member is relatively low, the height of the outer edge of the leg gather does not become too high. The opening for inserting the legs toward the outside in the width direction can be formed wider, thereby making it easier to pass the legs at the time of wearing.

When the disposable diaper illustrated in Fig. 7(a) is worn on the wearer, the state illustrated in Fig. 7(b) is obtained. When the wearer closes his/her legs, the leg gather bends convexly to the skin side. Specifically, it is bent with a folding line extending in the longitudinal direction as a base point, and the folding line becomes a vertex positioned on the skin side. The elongation rate of the inner leg elastic member 1632 positioned at the innermost side in the width direction among the plurality of leg elastic members is higher than the elongation rate of the leg elastic member positioned at the outer side of the inner leg elastic member in the width direction. Since the elongation rate of the inner leg elastic member is high, the leg gather 16 rises to the wearer side with the inner leg elastic member as the apex. In addition, since the elongation rate of the leg elastic member positioned on the outer side of the inner leg elastic member in the width direction is relatively low, it is possible to expand the wall by the leg gather from the inner leg elastic member toward the outside in the width direction. Since the wall with the inner leg elastic member as the apex can be formed on the outside in the width direction of the three-dimensional gather, side leakage of excrement can be prevented.

The length W16 of the leg gather in the width direction at the center of the disposable diaper in the longitudinal direction is longer than the distance W15 between the second base end portion 152 at the center of the disposable diaper in the longitudinal direction and the inner edge 15WI of the three-dimensional gather 15. The length of the leg gather in the width direction is the length of, in the width direction, the region including the nonwoven fabric sheet 161, the film sheet 162, and the leg gather elastic member 163 constituting the leg gather. The distance between the second base end portion 152 and the inner edge 15WI of the three-dimensional gather 15 is the rising height of the three-dimensional gather. At the center of the disposable diaper in the longitudinal direction, since the distance between the outer leg elastic member and the inner leg elastic member is longer than the distance between the second base end portion 152 and the inner edge 15WI of the three-dimensional gather 15, the wall by the leg gather 16 can be formed even when the three-dimensional gather falls to the outside in the width direction. Therefore, leakage of excrement can be suppressed by the leg gather while maintaining operability at the time of wearing.

(4) Absorber

Subsequently, the absorber 11 will be described in detail. A pair of lines 18 is provided in the absorber 11. The line 18 may be formed only on the absorbent core 11a, or may be formed on both the absorbent core 11a and the core wrap 11b. The pair of lines 18 is provided on both side portions of the absorber 11 in the width direction. The lines 18 are line-symmetric with respect to the center 1WC of the disposable diaper in the width direction as the axis of symmetry. Since the structure of both lines 18 is similar, one line will be described below.

In the line 18, a dent is provided on the skin side of the absorber, and a part in a squeezed state is provided on the non-skin side rather than the dent. Specifically, the line is formed by embossing. As illustrated in Fig. 2, the line 18 includes a first convex top portion 181, a second convex top portion 182, a third convex top portion 183, a rear side line 184, a front side line 185, and a center line 186. The first convex top portion 181 is a vertex portion protruding inward in the width direction. The first convex top portion 181 is positioned between the rear side line 184 and the center line 186 and is positioned at the center of the disposable diaper in the longitudinal direction or at the rear side of the center of the disposable diaper in the longitudinal direction. The bonded portion 153 is provided on a straight line passing through the first convex top portion 181 and extending in the width direction. In the region extending rearward from the first convex top portion 181, the absorber 11 deforms with the rear side line 184 as a base point and has a shape covering the buttocks widely. Since the bonded portion 153 is provided in a region having a shape that widely covers the buttocks, it becomes easier to make the three-dimensional gather 15 upright in the region covering the buttocks and leakage of excrement can be suppressed.

The rear side line 184 extends rearward from the first convex top portion 181 starting from the first convex top portion 181. The end point (rear end edge) of the rear side line 184 coincides with the outer edge of the absorbent core. The rear end edge of the rear side line 184 is positioned at the front side of the rear side exterior body. The rear side line 184 extends rearward from the center side of the disposable diaper in the longitudinal direction and extends outward in the width direction from the inside of the width direction. The outer edge of the rear side line is positioned at the outer edge of the absorbent core. The rear side line 184 extends rearward from the center side of the disposable diaper in the longitudinal direction and extends outward in the width direction from the inside of the width direction. Therefore, when a force directed from the outside of the width direction to the inside of the width direction is applied, it plays a role like a brace bar, and it is possible to suppress deformation of the absorber 11 inward in the width direction. Therefore, the absorber 11 is easily arranged along the swelling of the buttocks of the wearer. Since the absorber 11 is arranged so as to cover the swelling of the buttocks, the three-dimensional gather 15 is also likely to be arranged along the swelling of the buttocks, the three-dimensional gather 15 can be arranged at a more appropriate position, and leakage of excrement can be prevented by the three-dimensional gather 15.

In addition, the shape of the rear side line 184 in a plan view is a straight line shape extending to the outer side in the width direction with the first convex top portion 181 as a starting point and the rear side of the disposable diaper in the longitudinal direction, or a curved shape bent toward the outer side. Since it extends toward the outer side and the rear side in the width direction, it spreads outward in the width direction so as to cover the swelling of the buttocks from the crotch of the wearer toward the buttocks side. Therefore, the absorber 11 is easily arranged along the swelling of the buttocks of the wearer. Since the absorber 11 is arranged so as to cover the swelling of the buttocks, the three-dimensional gather 15 is also likely to be arranged along the swelling of the buttocks, the three-dimensional gather 15 can be arranged at a more appropriate position, and leakage of excrement can be prevented by the three-dimensional gather 15.

The second convex top portion 182 is a vertex portion protruding inward in the width direction. The second convex top portion 182 is positioned between the front side line 185 and the center line 186, and is positioned at the front side of the center of the disposable diaper in the longitudinal direction. The inner edge of the second convex top portion 182 is positioned outside the inner edge of the first convex top portion 181 in the width direction. The second convex top portion 182 is positioned at the rear side of the front side exterior body.

The front side line 185 extends frontward from the second convex top portion 182 starting from the second convex top portion 182. The end point (front end edge) of the front side line 185 coincides with the outer edge of the absorbent core. The front end edge of the front side line 185 is positioned in the region overlapping the front side exterior body. The front side line 185 extends frontward from the center side of the disposable diaper in the longitudinal direction and extends outward in the width direction from the inside of the width direction. The outer edge of the front side line 185 is positioned at the outer side of the outer side edge of the rear side line 184 in the width direction.

The third convex top portion 183 is a vertex portion protruding inward in the width direction. The third convex top portion 183 is positioned between the first convex top portion 181 and the second convex top portion 182, and is positioned at the front side of the center of the disposable diaper in the longitudinal direction. The inner edge of the third convex top portion 183 is positioned outside the inner edge of the first convex top portion 181 in the width direction. Therefore, the width of the absorber 11 is the narrowest in the portion where the first convex top portion 181 is positioned in the longitudinal direction.

The center line 186 is a line connecting the front side line 185 and the rear side line 184. The third convex top portion 183 is positioned at the central portion of the center line 186 in the longitudinal direction. The center line 186 includes a convex line protruding outward in the width direction between the first convex top portion 181 and the third convex top portion 183, and a convex line protruding outward in the width direction between the second convex top portion 182 and the third convex top portion 183.

A cutout 187 is provided on the outer edge of the absorber 11. The cutout 187 includes a first cutout 1871, a second cutout 1872, and a third cutout 1873. The position of the first cutout 1871 corresponds to the position of the first convex top portion 181 in the longitudinal direction. The position of the second cutout 1872 corresponds to the position of the second convex top portion 182 in the longitudinal direction. The position of the third cutout 1873 corresponds to the position of the third convex top portion 183 in the longitudinal direction.

The absorber 11 of the rear side region 2 has a high rigidity portion 19. The high rigidity portion 19 is a portion having higher rigidity than an absorber positioned at the center 1LC of the disposable diaper in the longitudinal direction. The rigidity indicates the difficulty of deformation of the absorber. Specifically, the high rigidity portion 19 is, for example, a portion having a width larger than the width of the absorber 11 positioned at the center 1LC of the disposable diaper in the longitudinal direction, a portion having a higher basis weight than the absorber 11 positioned at the center 1LC of the disposable diaper in the longitudinal direction, and a portion having high rigidity and having a line shape different from the absorber 11 positioned at the center 1LC of the disposable diaper in the longitudinal direction.

Since the rear side region 2 includes the high rigidity portion 19, the absorber in the rear side region is relatively high in rigidity. Even when it receives an elastic member positioned around the waist or a force directed inward in the width direction from both feet during wearing, it tends to repel in the width direction. Therefore, it is difficult for the left and right three-dimensional gathers 15 in the rear side region 2 to enter the inside of the width direction. Since it is difficult for the left and right three-dimensional gathers 15 to enter the inside of the width direction, the three-dimensional gather 15 can be positioned at an appropriate position so as to cover the swelling of the buttocks. Since it is difficult for the left and right three-dimensional gathers 15 to enter the inside of the width direction, it is possible to prevent the inner edge of the three-dimensional gather 15 from falling down so as to expand outward in the width direction when the three-dimensional gather 15 enters the inside. The three-dimensional gather 15 can be provided at a suitable position in the width direction and feces leakage can be suppressed by the three-dimensional gather 15.

The width of the absorbent core 11a in the rear side region is longer than the width of the absorbent core 11a positioned at the center 1LC of the disposable diaper in the longitudinal direction. In addition, the rigidity of the absorber 11 in the rear side region 2 may be higher than the rigidity of the absorber 11 in the front side region 3. The width of the absorbent core 11a in the rear side region may be longer than the width of the absorbent core 11a in the front side region. Specifically, the width W2 of the absorbent core in the rear side region illustrated in Fig. 4(b) and the width W3 of the absorbent core in the rear side region illustrated in Fig. 4(c) may be longer than the width W1 of the absorbent core in the rear side region illustrated in Fig. 4(a). Therefore, the rigidity of the absorbent core 11a in the rear side region is higher than the rigidity of the absorbent core 11a in the front side region. The width W3 of the absorbent core in the rear side region illustrated in Fig. 4(c) is longer than the width W2 of the absorbent core in the rear side region illustrated in Fig. 4(b). The width of the absorbent core 11a in the rear side region 2 is increased toward the rear side.

The high rigidity portion 19 of the rear side region 2 of the present embodiment is wider than the width of the absorber 11 positioned at the center 1LC of the disposable diaper in the longitudinal direction and includes the rear side line 184. In Fig. 2, the high rigidity portion 19 is indicated by hatching. The front end edge of the high rigidity portion 19 is positioned at the rear side of the center of the disposable diaper in the longitudinal direction and is positioned at the front side of the rear end edge of the bonded portion 153. The rear end edge of the high rigidity portion 19 is positioned at the front side of the rear side exterior body, and is positioned at the rear side of the rear end edge of the bonded portion 153.

Since the high rigidity portion 19 is wider than the width of the absorber 11 positioned at the center 1LC of the disposable diaper in the longitudinal direction, it is difficult to deform when a force directed from the outside in the width direction to the inside in the width direction is applied. In addition, the width of the rear side line 184 of the rear side region 2 is longer than the width of the center line 186 positioned at the center 1LC of the disposable diaper in the longitudinal direction. Therefore, the rear side region 2 is hardly deformed when a force directed from the outside in the width direction to the inside in the width direction is applied. Since the width of the absorber 11 is relatively wide and the width of the line extending outward in the width direction is relatively wide, the high rigidity portion 19 is hardly deformed when a force directed from the outside in the width direction to the inside in the width direction is applied.

The high rigidity portion 19 may be higher than the rigidity in at least a partial region of the front side region 3. In addition, the high rigidity portion 19 may be arranged in the entire rear side region 2, or may be arranged in a part of the rear side region 2. The rigidity of the absorber 11 can be measured by the following method. The absorbent having a size of 18 mm × 40 mm is used as a test sample. The measurement is performed by using a digital force gauge stand (FGP-5, manufactured by Nidec Shimpo Corporation). The tip of the measuring adapter has a linear shape. The test sample is placed on the measurement table of the digital force gauge stand. At this time, the test sample is placed so that the tip of the measuring adapter of the digital force gauge is parallel to the side of 18 mm and the tip of the measuring adapter is positioned at the center of the test sample. The test sample is pushed with the measuring adapter. The pushing speed is 1.5 mm/s. The maximum strength when pushed is set as a rigidity value. The absorber is hardly deformed as the rigidity value is higher.

In addition, the high rigidity portion 19 in the configuration in which the line (center line) is not provided in the absorber 11 positioned in the center 1LC of the disposable diaper in the longitudinal direction may be a portion having a longer width in the width direction than the absorber 11 positioned at the center 1LC of the disposable diaper in the longitudinal direction. The high rigidity portion 19 may be configured to be hardly deformed when a force directed inward in the width direction from the outside in the width direction is applied to the absorber 11 positioned at the center 1LC of the disposable diaper in the longitudinal direction.

(5) Other Embodiments

The bonded portion may not necessarily be arranged across the center of the disposable diaper in the longitudinal direction and may be arranged at least in the rear side region. The shape of the bonded portion in a plan view is not limited to a rectangle extending in the longitudinal direction, but may be a point shape spaced apart in the longitudinal direction. Due to the point shape of the bonded portion, it is possible to widen the accommodation space by the three-dimensional gather in the region between the bonded portions, thereby providing a wide accommodation space for excrement.

While the present invention has been described in detail with reference to the above-described embodiments, it is apparent to those skilled in the art that the present invention is not limited to the embodiments described in this specification. Furthermore, various changes and modifications can be made without departing from the spirit and scope of the invention as defined by the appended claims. Therefore, the descriptions of the present specification are intended for illustrative purposes and have no restrictive meaning for the present invention.

The entire contents of Japanese Patent Application No. 2016-35693, filed on February 26, 2016, as the basis of the priority claim of this application, are incorporated herein by reference.

[INDUSTRIAL APPLICABILITY]

According to the present disclosure, it is possible to provide a disposable diaper capable of securing an accommodation space for accommodating excrement and suppressing deterioration in operability at the time of wearing.

[REFERENCE SIGNS LIST]

| | |
|---|---|
| 1 | disposable diaper, |
| 2 | rear side region |
| 3 | front side region |
| 11 | absorber |
| 14 | side sheet (sheet member) |
| 15 | three-dimensional gather |
| 151 | first base end portion |
| 152 | second base end portion |
| 153 | bonded portion |
| 154 | upright portion |
| 155 | three-dimensional gather elastic member |
| 1551 | inner elastic member |
| 16 | leg gather |
| 161 | nonwoven sheet |
| 162 | film sheet |
| 163 | leg gather elastic member |
| 1631 | outer leg elastic member |
| 1632 | inner leg elastic member |
| L | longitudinal direction |
| T | thickness direction |
| T1 | skin side |
| T2 | non-skin side |
| W | width direction |

**Claims**

1. A pants-type disposable diaper (10), which has a longitudinal direction (L), a width direction (W) perpendicular to the longitudinal direction, and a thickness direction (T), the disposable diaper comprising:

   a front side region(3) positioned at a front side of a center (1LC) of the disposable diaper in the longitudinal direction, and a rear side region (2) positioned at a rear side of the center of the disposable diaper in the longitudinal direction;
   an absorber (11) arranged to straddle the front side region and the rear side region; and
   a pair of three-dimensional gathers (15) having a sheet member (14) and an elastic member (155) and arranged on both outer sides in the width direction with respect to the center of the disposable diaper in the width direction, wherein outer edges of the front side region and the outer edge of the rear side region are bonded,
   the three-dimensional gather comprises a base end portion incapable of being upright, and an upright portion (154) shrinkable in the longitudinal direction by the elastic member and capable of being upright on the skin side in the thickness direction with the base end portion as a base point,
   the base end portion comprises:

      a first base end portion (151) arranged at each of the front side region and the rear side region at both outer edges of the upright portion in the longitudinal direction; and
      a second base end portion (152) arranged at the outer edge of the upright portion in the width direction,

   the upright portion comprises a bonded portion (153) in which the sheet members are bonded to each other and a length of the upright portion in the width direction is narrowed,
   the bonded portion extends over the center of the disposable diaper in the longitudinal direction and is spaced from each of the first base end portions,
   a distance (L4) between the center of the disposable diaper in the longitudinal direction and the rear end edge of the bonded portion is longer than a distance (L5) between the center of the disposable diaper in the longitudinal direction and the front end edge of the bonded portion,
   the disposable diaper comprises a leg gather (16) arranged on the outer side of the three-dimensional gather in the width direction,
the leg gather comprises a plurality of leg elastic members (163) arranged at a distance in the width direction, and an elongation rate of the outer leg elastic member positioned on an outermost side in the width direction is lower than an elongation rate of the leg elastic member positioned at an inner side of the outer leg elastic member in the width direction.

2. The disposable diaper according to claim 1, wherein a distance (L6) between the first base end portion of the rear side region and the bonded portion is shorter than a distance (L7) between the first base end portion of the front side region and the bonded portion.

3. The disposable diaper according to claim 1 or 2, wherein a distance (L7) between the first base end portion in the front side region and the front end edge of the bonded portion is longer than a distance (L5) between the center of the disposable diaper in the longitudinal direction and the front end edge of the bonded portion.

4. The disposable diaper according to any one of claims 1 to 3, wherein a distance (L6) between the first base end portion in the rear side region and the rear end edge of the bonded portion is shorter than a total length of a length of the first base end portion in the rear side region in the longitudinal direction and a length of the bonded portion in the rear side region in the longitudinal direction and is longer than half the total length.

5. The disposable diaper according to claim 4, wherein an elongation rate of the inner leg elastic member (1632) positioned on an innermost side in the width direction is higher than an elongation rate of the leg elastic member positioned at an outer side of the inner leg elastic member in the width direction, and
a length of the leg gather in the width direction at the center of the disposable diaper in the longitudinal direction is longer than a distance between the second base end portion in the center of the disposable diaper in the longitudinal direction and the inner edge of the three-dimensional gather.

**Patentansprüche**

1. Höschenähnliche Einwegwindel (10), die Folgendes aufweist: eine Längsrichtung (L), eine Breitenrichtung (W), die senkrecht zu der Längsrichtung vorliegt, und eine Dickenrichtung (T), wobei die Einwegwindel Folgendes umfasst:

einen Vorderseitenbereich (3), der an einer Vorderseite einer Mitte (1LC) der Einwegwindel in der Längsrichtung positioniert ist, und einen Hinterseitenbereich (2),
der an einer Hinterseite der Mitte der Einwegwindel in der Längsrichtung positioniert ist;
einen Absorber (11), der zum Überspannen des Vorderseitenbereichs und des Hinterseitenbereichs angeordnet ist; und
ein Paar von dreidimensionalen Raffungen (15), die ein Lagenelement (14) und ein elastisches Element (155) aufweisen und an beiden äußeren Seiten in der Breitenrichtung in Bezug auf die Mitte der Einwegwindel in der Breitenrichtung angeordnet sind,
wobei äußere Ränder des Vorderseitenbereichs und der äußere Rand des Hinterseitenbereichs verbunden sind,
die dreidimensionale Raffung Folgendes umfasst: einen Basisendabschnitt, der nicht aufgerichtet werden kann, und einen aufgerichteten Abschnitt (154), der in der Längsrichtung durch das elastische Element schrumpfbar ist und auf der Hautseite in der Dickenrichtung mit dem Basisendabschnitt als Basispunkt aufgerichtet werden kann,
der Basisendabschnitt Folgendes umfasst:

einen ersten Basisendabschnitt (151), der an jedem des Vorderseitenbereichs und des Hinterseitenbereichs an beiden äußeren Rändern des aufgerichteten Abschnitts in der Längsrichtung angeordnet ist; und
einen zweiten Basisendabschnitt (152), der an dem äußeren Rand des aufgerichteten Abschnitts in der Breitenrichtung angeordnet ist,
der aufgerichtete Abschnitt einen gebundenen Abschnitt (153) umfasst, in dem die Lagenelemente miteinander verbunden sind und sich eine Länge des aufgerichteten Abschnitts in der Breitenrichtung verringert,
sich der gebundene Abschnitt über die Mitte der Einwegwindel in der Längsrichtung erstreckt und von jedem der ersten Basisendabschnitte beabstandet ist,
ein Abstand (L4) zwischen der Mitte der Einwegwindel in der Längsrichtung und dem hinteren Endrand des gebundenen Abschnitts länger ist als ein Abstand (L5) zwischen der Mitte der Einwegwindel in der Längsrichtung und dem vorderen Endrand des gebundenen Abschnitts,

die Einwegwindel eine Beinraffung (16) umfasst, die an der äußeren Seite der dreidimensionalen Raffung in der Breitenrichtung angeordnet ist,

die Beinraffung eine Vielzahl von elastischen Beinelementen (163) umfasst, die in einem Abstand in der Breitenrichtung angeordnet sind, und

die Dehnrate des äußeren elastischen Beinelements, das an einer äußersten Seite in der Breitenrichtung positioniert ist, geringer ist als eine Dehnrate des elastischen Beinelements, das an einer inneren Seite des äußeren elastischen Beinelements in der Breitenrichtung positioniert ist.

2. Einwegwindel nach Anspruch 1, wobei ein Abstand (L6) zwischen dem ersten Basisendabschnitt des Hinterseitenbereichs und dem gebundenen Abschnitt kürzer ist als ein Abstand (L7) zwischen dem ersten Basisendabschnitt des Vorderseitenbereichs und dem gebundenen Abschnitt.

3. Einwegwindel nach Anspruch 1 oder 2, wobei ein Abstand (L7) zwischen dem ersten Basisendabschnitt in dem Vorderseitenbereich und dem vorderen Endrand des gebundenen Abschnitts länger ist als ein Abstand (L5) zwischen der Mitte der Einwegwindel in der Längsrichtung und dem vorderen Endrand des gebundenen Abschnitts.

4. Einwegwindel nach einem der Ansprüche 1 bis 3, wobei ein Abstand (L6) zwischen dem ersten Basisendabschnitt in dem Hinterseitenbereich und dem hinteren Endrand des gebundenen Abschnitts kürzer ist als eine Gesamtlänge einer Länge des ersten Basisendabschnitts in dem Hinterseitenbereich in der Längsrichtung und einer Länge des gebundenen Abschnitts in dem Hinterseitenbereich in der Längsrichtung und länger ist als die Hälfte der Gesamtlänge.

5. Einwegwindel nach Anspruch 4, wobei eine Dehnrate des inneren elastischen Beinelements (1632), das an einer innersten Seite in der Breitenrichtung positioniert ist, höher ist als eine Dehnrate des elastischen Beinelements, das an einer äußeren Seite des inneren elastischen Beinelements in der Breitenrichtung positioniert ist, und eine Länge der Beinraffung in der Breitenrichtung an der Mitte der Einwegwindel in der Längsrichtung länger ist als ein Abstand zwischen dem zweiten Basisendabschnitt in der Mitte der Einwegwindel in der Längsrichtung und dem inneren Rand der dreidimensionalen Raffung.

## Revendications

1. Couche jetable de type culotte (10), qui a une direction allant dans le sens longitudinal (L), une direction allant dans le sens de la largeur (W) perpendiculaire par rapport à la direction allant dans la direction allant dans le sens longitudinal, et une direction allant dans le sens de l'épaisseur (T), la couche jetable comportant :

une région côté avant (3) positionnée au niveau d'un côté avant d'un centre (1LC) de la couche jetable dans la direction allant dans le sens longitudinal, et une région côté arrière (2) positionnée au niveau d'un côté arrière du centre de la couche jetable dans la direction allant dans le sens longitudinal ;
un élément absorbant (11) agencé pour enjamber la région côté avant et la région côté arrière ; et
une paire de fronces tridimensionnelles (15) ayant un élément formant feuille (14) et un élément élastique (155) et agencées sur les deux côtés extérieurs dans la direction allant dans le sens de la largeur par rapport au centre de la couche jetable dans la direction allant dans le sens de la largeur,
dans laquelle des bords extérieurs de la région côté avant et le bord extérieur de la région côté arrière sont liés,
la fronce tridimensionnelle comporte une partie d'extrémité de base incapable de se tenir à la verticale, et une partie verticale (154) rétrécissable dans la direction allant dans le sens longitudinal par l'élément élastique et capable de se tenir à la verticale sur le côté peau dans la direction allant dans le sens de l'épaisseur avec une partie d'extrémité de base tenant lieu de point de base,
la partie d'extrémité de base comporte :

une première partie d'extrémité de base (151) agencée au niveau de chacune parmi la région côté avant et la région côté arrière au niveau des deux bords extérieurs de la partie verticale dans la direction allant dans le sens longitudinal ; et
une deuxième partie d'extrémité de base (152) agencée au niveau du bord extérieur de la partie verticale dans la direction allant dans le sens de la largeur,
la partie verticale comporte une partie liée (153) dans laquelle les éléments formant feuille sont liés les uns par rapport aux autres et une longueur de la partie verticale dans la direction allant dans le sens de la largeur est rétrécie,

la partie liée s'étend sur le centre de la couche jetable dans la direction allant dans le sens longitudinal et est espacée par rapport à chacune des premières parties d'extrémité de base,

une distance (L4) entre le centre de la couche jetable dans la direction allant dans le sens longitudinal et le bord d'extrémité arrière de la partie liée est supérieure par rapport à une distance (L5) entre le centre de la couche jetable dans la direction allant dans le sens longitudinal et le bord d'extrémité avant de la partie liée,

la couche jetable comporte une fronce au niveau de la jambe (16) agencée sur le côté extérieur de la fronce tridimensionnelle dans la direction allant dans le sens de la largeur,

la fronce au niveau de la jambe comporte une pluralité d'éléments élastiques au niveau de la jambe (163) agencés à une distance dans la direction allant dans le sens de la largeur, et

un taux d'allongement de l'élément élastique au niveau de la jambe côté extérieur positionné sur un côté se trouvant le plus à l'extérieur dans la direction allant dans le sens de la largeur est inférieur par rapport à un taux d'allongement de l'élément élastique au niveau de la jambe positionné au niveau d'un côté intérieur de l'élément élastique au niveau de la jambe côté extérieur dans la direction allant dans le sens de la largeur.

2. Couche jetable selon la revendication 1, dans laquelle une distance (L6) entre la première partie d'extrémité de base de la région côté arrière et la partie liée est inférieure par rapport à une distance (L7) entre la première partie d'extrémité de base de la région côté avant et la partie liée.

3. Couche jetable selon la revendication 1 ou la revendication 2, dans laquelle une distance (L7) entre la première partie d'extrémité de base dans la région côté avant et le bord d'extrémité avant de la partie liée est supérieure à une distance (L5) entre le centre de la couche jetable dans la direction allant dans le sens longitudinal et le bord d'extrémité avant de la partie liée.

4. Couche jetable selon l'une quelconque des revendications 1 à 3, dans laquelle une distance (L6) entre la première partie d'extrémité de base dans la région côté arrière et le bord d'extrémité arrière de la partie liée est inférieure à une longueur totale d'une longueur de la première partie d'extrémité de base dans la région côté arrière dans la direction allant dans le sens longitudinal et d'une longueur de la partie liée dans la région côté arrière dans la direction allant dans le sens longitudinal et est supérieure à la moitié de la longueur totale.

5. Couche jetable selon la revendication 4, dans laquelle un taux d'allongement de l'élément élastique au niveau de la jambe côté intérieur (1632) positionné sur un côté se trouvant le plus à l'intérieur dans la direction allant dans le sens de la largeur est supérieur à un taux d'allongement de l'élément élastique au niveau de la jambe positionné au niveau d'un côté extérieur de l'élément élastique au niveau de la jambe côté intérieur dans la direction allant dans le sens de la largeur, et

une longueur de la fronce au niveau de la jambe dans la direction allant dans le sens de la largeur au niveau du centre de la couche jetable dans la direction allant dans le sens longitudinal est supérieure à une distance entre la deuxième partie d'extrémité de base dans le centre de la couche jetable dans la direction allant dans le sens longitudinal et le bord intérieur de la fronce tridimensionnelle.

# FIG. 1

# FIG. 2

# FIG. 3

# FIG. 4

(a)

(b)

(c)

# FIG. 5

(a)

(b)

# FIG. 6

(a)

(b)

EP 3 406 232 B1

# FIG. 7

(a)

(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014180346 A

- JP 2016035693 A